# EUROPEAN PATENT SPECIFICATION

(11) **EP 3 749 348 B1**
(45) Date of publication and mention of the grant of the patent: **17.04.2024**
(21) Application number: 19702105.8
(22) Date of filing: 05.02.2019
(51) Int. Cl.: A61K 38/40, A61K 36/185, A61P 11/00, A61P 37/04

(54) **COMPOSITION FOR THE PREVENTION AND TREATMENT OF DISEASES OF THE RESPIRATORY TRACT**
ZUSAMMENSETZUNG ZUR PRÄVENTION UND BEHANDLUNG VON ATEMWEGSERKRANKUNGEN
COMPOSITION POUR LA PREVENTION ET LE TRAITEMENT DE MALADIES RESPIRATOIRES

(30) Priority: 07.02.2018 IT 201800002457
(43) Date of publication of application: 16.12.2020
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Longoni, Alessandra
(86) International application number: PCT/EP2019/052696
(87) International publication number: WO 2019/154773

(56) References cited:
- EP-A1- 2 481 751
- WO-A1-2014/060529
- WO-A1-2017/126843
- WO-A2-03/099207
- KR-A- 20160 081 359
- US-A1- 2007 253 941

## Description

The present invention relates to a composition containing an association of an extract of *Pelargonium sidoides* and lactoferrin useful for the prevention and/or treatment of diseases of the respiratory tract and for use as stimulant of the immune system. The composition is particularly effective as it allows to obtain at the same time an antiviral effect, an antimicrobial effect and an immunomodulatory effect thanks to the synergistic action of its components.

### Background of the invention

### RESPIRATORY TRACT AND RESPIRATORY DISEASES

The respiratory tract is composed of different anatomical structures that serve to the correct function thereof.

It is, in fact, intended for the gas, oxygen and carbonic anhydride exchange, between tissues and external environment which is fundamental for all the cellular processes of the organism and that is for its life.

Anatomically two macro areas which are upper and lower airways can be distinguished. The first ones are composed of nose, pharynx and associated structures, while the second ones are composed of larynx, trachea, bronchi and lungs, whose real respiratory surface is made by alveoli. This complex system of organs serves to prepare the air to its entry in lungs, through the filtration from potential particulates, warming and moistening. At the level of nasal cavities particles bigger than 10-15 microns can be blocked, this thanks to vibrissae and to the presence of mucus that entraps these particles besides turbulence precipitation, mediated by turbinates which deviate the air direction. Mucus stratifies on periciliary liquid, in which are immersed the vibratile cilia of epithelial cells. Particles of about 10 microns reach the trachea, are trapped in the mucus and then eliminated by ciliary movement. Particles of 2-5 microns sediment in terminal bronchioles by gravitational precipitation, while those of dimensions less than 2 microns are removed by alveolar macrophages and moved away by lymphatic pulmonary system.

The whole respiratory tract is composed of epithelial cells differing in type and function along the tracheobronchial tree. Ciliated columnar cells characterize the airways from trachea to terminal bronchioles. From their apical surface protrude cilia that have the task of moving with a cleaning effect on mucus and potentially inhaled particles. Globet cells have the task of secreting mucus, useful to maintain the correct moistening of the epithelium and to entrap particulate. They are present in the largest tracts of the airways under the small bronchi but they have not been found in bronchioles.

All the components of the respiratory tract can be exposed to a series of diseases, of different aetiology, often sustained by different pathogenic microorganisms which, becoming preponderant in the microenvironment and on the normally present flora, determine the disease and, as a consequence, leads to a reduced functionality.

### EXAMPLES OF RESPIRATORY TRACT DISEASES

Depending on the area of the respiratory tract several pathological conditions can be distinguished characterised by inflammations due to viruses, bacteria or fungi.

### Cold and flu

Cold (also referred as "*common cold*") is an acute and self-limiting infection of the upper airways which involves the nose, nasal cavities, pharynx and larynx. The virus is transmitted by contact with an infected person through air, generally through coughs and sneezes or direct contact with the virus. The incubation period normally is less than two days and the symptoms usually have a peak between the first and third day with a variable duration between 7 and 10 days. The most common symptoms are sore throat, rhinitis, rhinorrhoea, cough and general disconfort and the severity of the symptoms varies greatly from person to person and depending on the different infective agents. For example, the fever is usually infrequent in adults but very frequent in children and in general the incidence of cold syndromes decreases with the age. In fact, children under two years of age tend to have up to six episodes per year, the adults between two and three while the elderly about one episode per year. In children triggering causes may be the daily attendance of kindergartens which increases the contagion risk while for the adults stress and sleep problems can favour the onset of cold. *Rhinovirus* are mainly involved in these type of infections, in other cases it is not well known which the etiological agent is while only in 5% of cases they are bacterial infections. Although it is a disorder which tends to resolve spontaneously, the cold has a very high prevalence and can be debilitating as it can determine, for example, a reduction of the productivity at work and interfere with other activities such as driving. Furthermore, it has a high impact on health; in fact about 7-17% of adult people and 33% of children require medical consultation. For example, in the United States the cost related to cold (medical examination, superinfection and drugs) has been estimated of 17 billion of dollars in 1997.

In the case of flu it is always a viral infection that deeply affects the world population mainly between the months of December and April. Unlike the cold, it is accompanied by fever, headache, myalgia, lethargy and loss of appetite.

### Rhinitis

Rhinitis is an inflammatory process which affects the mucosa of nasal cavities and is divided in acute and chronic. Acute rhinitis are generally sustained by viruses, among which *Rhinovirus, Coronavirus,* influenza and parainfluenza viruses, RSV, *Coxsackie virus,* ECHO virus and adenovirus. The contagion occurs by direct contact with the ill subject presenting, in the peak of maximum contagiousness (generally the first day), 500-1000 virions per ml of secrete, that he emits through caught and sneezes. Bacterial superinfections are possible which lead to complications such as otitis and sinusitis. The common cold by *Rhinovirus* determines an acute symptomatology in the first 3-4 days, while for 7-10 days caught and other symptoms persist. There is an excess of mucous secretions that are fluid and transparent, and become purulent and malodorous in case of bacterial superimposition.

The chronic form is generally secondary to sinusitis, nasal septum deviations, and hypertrophic adenoids.

Allergic rhinitis is due to the exposure by the subject to substances that causes him an IgE mediated reaction, characterised by excessive production of liquids, intranasal itching, sneezes and obstructions. IgEs, in fact, bind to mast cells which release large amounts of histamine, responsible of all morbid manifestations.

Recent studies highlighted that allergic rhinitis and asthma are concurrent and to be considered as two manifestations of the whole respiratory tract, rather than locate them one in the superior tract and the other in the inferior tract of the respiratory tree. In fact it appears that between 20% and 50% of patients with allergic rhinitis have also asthma and that from 30% to 90% of patients with asthma have concomitant rhinitis. Therefore, allergic rhinitis could be a predisposing factor to the development of allergic asthma, and specifically the sensitisation to allergens (pollen or animal hair) would seem an important risk factor in the association of asthma and rhinitis.

### Sinusitis

Sinusitis is the inflammation of the mucosae covering the nasal cavities, bone cavities located in the facial grouping of bones, and which are in communication with nasal cavities, and therefore can become infected for the same causes determining rhinitis. Sinusitis can be divided in acute viral or acute bacterial (up to 4 weeks), chronic (beyond 12 weeks) and acute recurrent (at least 4 episodes per year with resolution). When sinusitis involves the nasal cavity is called rhinosinusitis. Generally, a healthy sinus is sterile, characterised by suitable mucus drainage and free passage of the air. Abnormality or ciliary immobility determine the inhibition of the drainage resulting in sinusitis. Predisposing factors to this disease are immunocompromised state, nasal septum deviation, nasal polyps, tumours, trauma and fractures, cocaine abuse, presence of foreign bodies.

An acute viral form can be subjected to bacterial superinfection. Bacteria generally responsible of these infections are *Streptococcus pneumoniae,* nontypeable *Haemophilus influenzae, Moraxella catarrhalis. Pseudomonas aeruginosa* is more frequently present in sinusitis by HIV infection and cystic fibrosis. Some genera of fungi such as *Candida, Aspergillus, Blastomyces, Coccidioides, Rizophus, Histoplasma* and *Cryptococcus* can cause sinusitis in immunocompromised patients. Signs and symptoms of acute rhinosinusitis consist in: mucopurulent leaks from the nose, nasal obstruction, congestion, facial pain, pressure to the involved sinuses, hyposmia, anosmia, fever, sensation of pressure or "plug" to ears, dental pain. Generally in the first 3-5 days it is not possible to distinguish a viral form from a bacterial form, therefore the use of antibiotics is not recommended. If the disease persists beyond 10 days then it is very likely sustained by bacteria and the antibiotic treatment is indicated. Chronic forms have a slower onset, greater duration and frequency. Symptoms are similar to those of the acute form with, in addition, bad breath, laryngitis, bronchitis and asthma worsening.

Sinusitis often resolves spontaneously and the treatment is prevalently symptomatic. In particular, the decongestant treatment serves to reduce oedema, ameliorating the drainage of mucus in excess and maintain the patency of sinus ostia. A good result can be obtained from the local application of hypertonic saline solution both in the treatment of acute bacterial or acute recurrent form and chronic form and also in prevention.

The selection of antibiotic instead has to take into consideration the production of beta-lactamase and the presence of drug resistant pneumococci.

### Pharyngitis (pharyngotonsillitis)

It's an inflammatory process of pharynx, hypopharynx, uvula and tonsils, which is generally transmitted by direct contact with respiratory secretions. It is more frequent in children (5-15 years) and, although is frequently self-limiting, the swelling of the involved parts can cause a reduced patency of the airways or, anyway, preclude the ingestion of adequate amounts of liquids with consequent dehydration.

The infection can be sustained by viruses (such as Epstein-Barr) and bacteria, in particular beta-haemolytic *Streptococcus pyogenes* of group A is the most frequent in pediatric forms, but also *Mycoplasma pneumoniae* and *Chlamydia pneumoniae* are found in adults and children. Moreover, forms transmitted by sexual contact and sustained by *Neisseria gonorrhoeae* and those by *Corynebacterium diphtheriae* (form reduced by the use of the vaccine) are to be considered.

### Epiglottitis

It is an inflammation of the epiglottis, consequent to a viral or bacterial infection, determining swelling of the organ with possible obstruction of the airways.

It is prevalently caused by type b *H. influenzae,* but also by streptococci, staphylococci or a thermic trauma. It manifests itself with hears pain (in adults), dysphonia, while fever is absent up to 50% of cases and can develop in a tardive phase. The treatment is antibiotic when bacteria are the cause of the disease, while intubation can be requested in cases of severe obstruction of the airways.

### Laryngitis

Larynx inflammation is manifested by aphonia and hoarseness, mainly caused by viruses, but up to 10% of cases also by bacteria (streptococci and *C*. *diphtheriae* included). Non infective causes can be tumours, thermic or caustic trauma, gastroesophageal reflux disease (GERD). Laryngitis presents symptoms which last 3-4 days and, unless the presence of bacteria, antibiotics are not used.

### Bronchitis

Bronchitis is a frequent inflammation of bronchi that is manifested by caught, shortness of breath and chest pain. Bronchitis is divided in acute and chronic; in the first case caught lasts about three weeks and in 90% of cases is caused by viral infection that can occur after contagion with other infected persons. Predisposing risk factors can be cigarette smoke or other sources of environmental pollution. Chronic bronchitis is characterised by caught for three months per year for at least two episodes per year and cigarette smoke together with others genetic or environmental causes are the main triggering factors. Normally, except in certain particular cases, antibiotics are not used but steroidal anti-inflammatory drugs, paracetamol in order to obviate the potential increase of temperature and bronchodilators such as salbutamol to ameliorate the respiration.

### Bronchiolitis

Bronchiolitis, a frequently pediatric illness, is characterized by an extensive inflammation of the airways accompanied by an intense production of mucus and necrosis of epithelial cells. It is mainly caused by a viral infection, in particular by *Respiratory Syncytial Virus (RSV),* but also by adenovirus, influenza and parainfluenza viruses, human metapneumovirus and rhinovirus, while the most frequently involved bacteria are of the genus *Chlamydia.*

In pediatric age the main clinical manifestations are tachypnea, breathlessness or crackles during auscultation, which generally follow an infection to the upper airways. The treatment may provide for hospitalization in case that the oxygen saturation is comprised between 92% and 94%, together with other clinical manifestations such as poor nutrition, dehydration and a history of dyspnoea.

### Bronchiectasis

It is a disease characterized by an irreversible dilatation of a portion of the bronchial tree in the lungs. The bronchial dilatation can be the result of a structural defect of the wall, the exposure to an abnormal pressure or a cartilage or elastic tissue damage due to an inflammation. It involves bronchi and bronchioles wherein a vicious circle of infection and inflammation, also with the release of mediators, can be established. Common symptoms are cough productive of mucus and chest pain. The mucus presents an increased quantity of elastase and TNF-α, IL-8 and prostanoids. It can be presented as a local obstructive process or widespread part of both lobes, accompanied also by sinusitis or asthma. The causes are various, for example, especially in pediatric age, infection also fungal which leaves a permanent damage. Otherwise the primary ciliary dyskinesia, in which there is a marked retention of secretions followed by infections. Cystic fibrosis, as well as immunodeficiency conditions can be predisposing factors. It was also observed that in patients with ulcerative colitis infections of the respiratory tract and bronchiectasis are present. The treatment involves the use of antimicrobials to fight infections sustained as much by bacteria as by fungi. Moreover, it is particularly useful to wash the airways to increase the removal of secretions, using saline solutions and maintain the patient, generally, well hydrated.

It was now surprisingly found that an association of an extract of *Pelargonium sidoides* and lactoferrin is particularly effective in the prevention and treatment of respiratory diseases and for the stimulation of the immune system thanks to the synergistic action of its components.

In all these above-mentioned diseases, as well as in other pathological conditions, is necessary to have available active ingredients able to activate in a significant manner the immune system in order to contrast in an effective and rapid way the infection while reducing the duration and the severity of the symptoms.

### Detailed description of the invention

Therefore an object of the present invention is a composition containing an association of an extract of *Pelargonium sidoides* and lactoferrin for the prevention and the treatment of respiratory diseases and for stimulation of the immune system. The components of the association according to the present invention are all known components, widely used in therapy.

### PELARGONIUM SIDOIDES

*Pelargonium sidoides* is one the most important species of the genus *Pelargonium* which have been used for a long time in traditional medicine of South Africa.

The popular use of this type of plant was aimed to respiratory infections and gastrointestinal problems; in particular the interest in this plant species has grown more and more for its potential use as anti-tuberculosis, as remedy for earache, cold, tonsillitis, bronchitis, sinusitis and rhinopharyngitis.

From a phytochemical point of view numerous studies have been directed to the identification of the main metabolites in the plant extracts and the presence of a large number of metabolites belonging to the classes of coumarins, flavonoids, proanthocyanidins, phenolic acids and phenylpropanoids has emerged. In particular, the "umcalin" and other trimethoxy-coumarins are considered as markers of the *Pelargonium sidoides* specie, to distinguish it for example from *Pelargonium reniforme* specie, in which these compounds should be absent.

The effects scientifically recognised to *P. sidoides* are the antibacterial, antiviral and immunomodulatory ones which have been attributed to polyphenols (gallic acid) and to a combination of phenolic compounds and compounds with coumarin structure respectively.

As regards the antibacterial and antifungal activity a 1997 study evaluated the effect of the individual components (scopoletin, "umcalin", 5,6,7-trimethoxy-coumarin, catechin-(+), gallic acid and esters thereof) against Gram positive bacteria (*Staphylococcus aureus, Streptococcus pneumoniae, Streptococcus* 1451) and Gram negative (*Escherichia coli, Klebsiella pneumoniae, Proteus mirabilis, Pseudomonas aeruginosa, Haemophilus influenzae*)*.* All the components except for catechin have shown antibacterial activity with a MIC comprised between 200 and 1000 µg/mL. The antibacterial activity was evaluated also indirectly by evaluating the ability of an extract of *Pelargonium sidoides* to inhibit the bacterial adhesion (streptococcus) to the human epithelial cells. Other studies *in vitro* have shown for the extract of the plant the ability of increasing phagocytosis, oxidative response and cellular death.

Other studies confirmed the significant immunomodulatory power of the extracts of *Pelargonium* able to stimulate, in addition to the macrophages activity, also the release of a series of cytokines essential to the immune response such as TNF-α, iNOS, IL-1, IL-10, IL-12, IL-18, interferon-α,γ. This immunomodulatory action is of prime importance considering also that the most of upper airways infections are due to viruses.

A 2011 study of Janecki and collaborators, highlighted a potential role of procyanidins contained in the root of *P. sidoides* in the inhibition of *Streptococcus pyogenes* adhesion to epithelial cells of the larynx.

Another documented activity of the plant extract is the ability to stimulate the mucociliary system: in two studies the ability of the plant extract (1, 30, 100 µg/mL) to increase the mucociliary clearance in cell culture of human nasal epithelium has been observed.

The action of *Pelargonium sidoides* has been evaluated in humans in about 20 clinical studies most of which was of a randomized type, double blind and placebo-controlled. In the studies conducted, the extract turned out to be able to reduce the severity and the duration of acute bronchitis, tonsillopharyngitis and other infections of the respiratory tract in adults and children.

For example in a 2006 study 400 patients between 6 and 18 years old were recruited which in a randomized manner received 30, 60 and 90 mg of extract of *Pelargonium sidoides* divided in three daily administrations or the placebo for a period of 7 days.

After 7 days the authors describe an improvement in the *Bronchitis Severity Score* (BSS) at dosages of 60 and 90 mg/die and in particular it also improved cough, wheezes during auscultation and *sputum.* In treated groups the tolerance of the product was comparable to that of the placebo.

For tonsillopharyngitis, among others, a study on 143 children between 6 and 10 years of age with a diagnosis of acute tonsillopharyngitis and a *Tonsillopharyngitis Severity Score* (TSS) ≥ 8 was conducted.

To children 1 mL of extract of *P*. *sidoides* EPs 7630 was administrated three times a day or the placebo for a duration of six days. In the treated group a TTS reduction significantly superior with respect to the placebo has been recorded, a lower use of antipyretic drugs without particular side effects.

In a 2007 study, the effectiveness of the treatment of cold symptoms with the extract of *P*. *sidoides* EPs 7630 was observed on 133 patients taking it for a maximum duration of ten days.

Also for common cold the extract turned out to be an effective treatment able to reduce the severity and duration of symptoms with respect to the placebo.

For the wide number of clinical trials from which a good effectiveness and, at the same time, a good safety of use (mainly slight and transitory side effects) emerged, the extract of *Pelargonium sidoides* represents a valid choice for the development of a product for the prevention and treatment of diseases of the respiratory apparatus.

### LACTOFERRIN

Lactoferrin (Lf), formally known also with the name of lactotransferrin, is a glycoprotein able to bind iron belonging to the family of transferrin together with serum transferrin, ovotransferrin and melanotransferrin.

Lactoferrin was isolated in 1939 from bovine milk and after several years has been found that it was the main protein of human milk able to bind iron. Lf is produced and released by epithelial cells from mucosae and neutrophils of different species of mammals among which human, bovines, horses, dogs and several rodents. Several studies have shown how such compound is involved in different physiological and protective functions such as the regulation of iron absorption in the intestine as well as how it is endowed with antioxidants, anti-inflammatory, antimicrobial and anticancer properties. In milk and colostrum lactoferrin is present in a concentration of 7g/L and it is highly concentrated also within saliva, sweat, vaginal fluids, sperm, bronchial, nasal, biliary secretions and in urine.

From a structural-chemical point of view lactoferrin is a glycosylated protein of 80kDa containing 700 amino acids with an elevated homology between different species. Its three-dimensional structure consists of a single polypeptide chain wrapped in two symmetrical lobes (lobes N and C) which are very similar to each other.

In addition to iron, lactoferrin is able to bind also copper, zinc and manganese ions and in biological fluids it can be present both in an iron-free (apo-lactoferrin) or iron-bound (olo-lactoferrin) form. Despite being a protein, scientific works report the ability of bovine lactoferrin (approved for the use in food supplements) to withstand the passage in the gastric environment upon oral administration. Despite a partial digestion of the molecule is plausible from such a reaction cationic peptides are formed which show comparable if not higher activities with respect to the native protein such as in the case of lactoferricin peptide.

One of the main activity of lactoferrin is the antibacterial one: such compound in fact is very effective with both Gram positive and Gram negative bacteria. Its effectiveness is due to a double mechanism of action, one depending on its ability to bind iron and one independent. The high ability to bind iron determines a poor availability of this particularly important metal to certain bacterial species for adhesion and proliferation. Examples of of this type of pathogens are *E.coli, Pseudomonas aeruginosa, Burkholderia cepacia* for which lactoferrin through this mechanism is able to reduce the adhesion to mucosae and the biofilm formation.

The other mechanism of action is linked to its ability to bind the lipopolysaccharide (LPS) present in the plasmatic membrane of Gram negative bacteria and lipotecoic acid present in the membrane of Gram positive. On the surface of bacteria receptors for the N-terminal portion of lactoferrin have been identified, such bond determines a structural alteration of LPS and lipotecoic acid entailing an increase in the permeability of the bacterial membrane.

Moreover, lactoferrin is endowed with a marked antiviral activity against different viruses such as Cytomegalovirus, Herpes simplex virus 1 and 2, human immunodeficiency virus (HIV), hepatitis C virus, papilloma virus. As regards the present invention of particular importance are the activities against the *Respiratory Syncytial Virus* (RSV) where it interacts with F protein the main glycoprotein involved in cell invasion, against the parainfluenza Virus (PIV) where it inhibits the virus adhesion preventing invasion and replication and against the Adenovirus where it binds the polypeptide III responsible for the virus adhesion through integrins to host cell surface. Lactoferrin is particularly active also against influenza A virus where it inhibits cell death interfering with caspase 3 which is the main effector induced by the virus and furthermore it stops the export from the cell nucleus of viral ribonucleotides thus preventing the assembly of the virus within the host cell.

Lactoferrin has a significant immunomodulatory power due to both an anti-inflammatory activity and its ability to activate innate and acquired immune response. Specifically, Lf interacts with surface receptors expressed by antigen-presenting cells (APC) such as monocytes, macrophages, dendritic cells, neutrophils by inducing the activation and migration thereof in the site of infection. Moreover it increases the expression of adhesion molecules such as Beta1, Beta2, ICAM-1 on the surface of monocytes, factors stimulating the synthesis of granulocytes and monocytes extending their action in the site of interest and it increases also the expression of CD80, CD83, CD86 glycoproteins with an increase of pro-inflammatory cytokines production.

The antibacterial, antiviral and immunomodulatory effectiveness of lactoferrin makes it a valid active ingredient to be used for the prevention and treatment of infections of the respiratory system.

In a preferred embodiment, the composition object of the present invention contains the association of an extract of *Pelargonium sidoides* and lactoferrin in admixture with a suitable acceptable carrier.

Suitable acceptable carriers are those commonly known to the man skilled in the art for the preparation of compositions for oral administration such as solutions, suspensions, powders or granulates, tablets, capsules, pellets.

By way of non-limiting example, said acceptable carriers can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners.

Specifically examples of diluents can be: magnesium carbonate, cellulose microcrystalline, starch, lactose, and sucrose; mainly used lubricants are magnesium stearate, stearic acid, and sodium stearyl fumarate. As glidants colloidal silica and magnesium silicate, as disintegrants the cross-linked polyvinylpyrrolidones, and sodium starch glycolate, as solubilizing agents surfactants such as TWEEN or sodium lauryl sulphate, and as stabilizers all classes of preservatives (sorbic acid and derivatives, benzoic acid and derivatives, parabens), antioxidants (ascorbic acid and derivatives, tocopherol), and acidifying agents (phosphoric acid, tartaric acid) can be cited. Thickeners and gelling agents can be carrageenan, pectins, and starches, coating agents include for example waxes and derivatives, anti-caking agents include for example calcium or magnesium carbonate, humectants include for example sorbitol and mannitol, sequestrants include for example EDTA and derivatives, sweeteners include for example aspartame, and acesulfame potassium.

The composition object of the present invention is preferably a liquid or solid composition for oral use, still more preferably an aqueous solution, a suspension, a powder or granulate, a tablet, a capsule, and pellet.

The composition object of the present invention contains extract of *Pelargonium sidoides* and lactoferrin.

The extract of *Pelargonium sidoides* is present in an amount between 1 mg and 1000 mg, preferably between 10 mg and 500 mg, still more preferably between 20 mg and 200 mg.

Lactoferrin is present in an amount between 1 mg and 1000 mg, preferably between 10 mg and 500 mg, still more preferably between 20 mg and 200 mg.

The compositions object of the present invention are particularly effective in the prevention and/or treatment of diseases of the respiratory tract, in particular in the prevention and/or treatment of rhinitis, allergic rhinitis, rhinopharyngitis or cold, rhinosinusitis, sinusitis, pharyngitis, epiglottitis, laryngitis, bronchitis, bronchiolitis and bronchiectasis allowing to obtain at the same time an antiviral effect, an antimicrobial effect and an immunomodulatory effect thanks to the synergistic action of their components.

Thanks to the properties of the active ingredients the composition object of the present invention is particularly effective as a stimulant of the immune system.

Therefore another object of the present invention is a composition containing the association of an extract of *Pelargonium sidoides* and lactoferrin in admixture with a suitable acceptable carrier for use in the prevention and/or treatment of diseases of the respiratory tract and for the stimulation of the immune system.

The composition can be a medical device, a food supplement, a nutraceutical, dietetic and nutritional composition, a food product, a beverage, a nutraceutical product, a medicament, a medicated food, a pharmaceutical composition or a food for special medical purposes.

Without being bound to a specific theory, the inventors are of the opinion that the synergistic effect of the association present in the composition object of the present invention, derives from the following activities of the components of the association. The extract of *Pelagornium sidoides* has a marked antibacterial, antiviral and immunomodulatory activity thanks to the ability of its components to prevent the bacterial adhesion, stimulate the phagocytosis and the release of various pro-inflammatory cytokines (interleukins, interferon γ and TNF-α, iNOS); moreover it is able to stimulate the mucociliary clearance resulting effective in most of the affections at a respiratory level.

Lactoferrin has antibacterial activity with a unique mechanism of action linked to its high ability to sequester iron from bacterial cells causing the death thereof; moreover it is able to bind lipopolysaccharide and lipotecoic acid, two important components of the membrane of Gram negative and Gram positive bacteria. Lactoferrin is particularly effective also against common viruses such as the influenza or cold virus thanks to its ability to interfere with cell death through an inhibiting action on caspase 3. Finally, by interacting with surface receptors of antigen-presenting cells, it is able to induce cell activation and migration in the site of infection.

The synergistic activity of the above-mentioned active ingredients can be studied by *in vitro* and/or *in vivo* tests able to assess the antiviral, antimicrobial and immunomodulatory activity of the compositions according to the present invention and/or of the comparative compositions.

For the evaluation of the antiviral, antimicrobial and immunomodulatory activity any test known in the literature can be used.

As an indication, the antiviral activity for example is evaluated through different type of tests which allow to evaluate the effect of the tested compounds on the virus plaques, on a particular effect (cytotoxicity) thereof, on certain proteins essential for viruses, or on particular phases of the reproductive cycle such as attachment, entry, uncoating, replication, assembly, release etc.

The antimicrobial activity instead is evaluated on the main bacterial strains belonging to Gram positive and/or Gram negative categories and/or other microbial species. As regards the immunomodulatory activity, it is preferably evaluated through scientific tests able to detect the stimulation/inhibition ability of cells, cytokines or of other factors involved in the immune response.

### Examples

By way of example are now provided some non-binding examples of daily doses of active components of the composition object of the present invention.

Daily doses are meant to be administrated in a suitable oral dosage form and divided in one or more dosage units.

### EXAMPLE 1 (coated tablet)

| Active ingredient | Daily dose |
|---|---|
| *Pelargonium sidoides* extract | 60 mg |
| Lactoferrin | 100 mg |

### EXAMPLE 2 (powder or granulate for oral suspension/solution)

| Active ingredient | Daily dose |
|---|---|
| *Pelargonium sidoides* extract | 120 mg |
| Lactoferrin | 200 mg |

### EXAMPLE 3 (powder or granulate for oral suspension/solution)

| Active ingredient | Daily dose |
|---|---|
| *Pelargonium sidoides* extract | 30 mg |
| Lactoferrin | 200 mg |

### EXAMPLE 4 (capsule)

| Active ingredient | Daily dose |
|---|---|
| *Pelargonium sidoides* extract | 20 mg |
| Lactoferrin | 20 mg |

### EXAMPLE 5 (liquid flacon for oral use)

| Active ingredient | Daily dose |
|---|---|
| *Pelargonium sidoides* extract | 40 mg |
| Lactoferrin | 80 mg |

The compositions are prepared according to conventional techniques such as mixing.

## Claims

1. A composition containing an association of an extract of *Pelargonium sidoides* and lactoferrin in admixture with a suitable acceptable carrier.

2. A composition according to claim 1 in the form of a liquid or solid composition for oral use.

3. A composition according to claim 1 or 2 wherein the extract of *Pelargonium sidoides* is present in an amount between 1 mg and 1000 mg, preferably between 10 mg and 500 mg, still more preferably between 20 mg and 200 mg.

4. A composition according to any of the previous claims wherein lactoferrin is present in an amount between 1 mg and 1000 mg, preferably between 10 mg and 500 mg, still more preferably between 20 mg and 200 mg.

5. Composition according to any of the previous claims wherein the composition is a medical device, a food supplement, a nutraceutical, dietetic and nutritional composition, a food product, a beverage, a nutraceutical product, a medicament, a medicated food, a pharmaceutical composition or a food for special medical purposes.

6. A composition according to any of the previous claims for use in the prevention or treatment of diseases of the respiratory tract.

7. A composition according to claim 6 for use in the prevention or treatment of rhinitis, allergic rhinitis, rhinopharyngitis or cold, rhinosinusitis, sinusitis, pharyngitis, epiglottitis, laryngitis, bronchitis, bronchiolitis and bronchiectasis.

8. A composition according to any of the claims 1 to 5 for use as stimulant of the immune system.

## Patentansprüche

1. Zusammensetzung, die eine Assoziation aus einem Extrakt aus *Pelargonium sidoides* und Lactoferrin in Beimischung mit einem geeigneten akzeptablen Träger enthält.

2. Zusammensetzung nach Anspruch 1 in der Form einer flüssigen oder festen Zusammensetzung zur oralen Verwendung.

3. Zusammensetzung nach Anspruch 1 oder 2, wobei das Extrakt aus *Pelargonium sidoides* in einer Menge zwischen 1 mg und 1000 mg vorhanden ist, bevorzugt zwischen 10 mg und 500 mg, noch stärker bevorzugt zwischen 20 mg und 200 mg.

4. Zusammensetzung nach einem der vorhergehenden Ansprüche, wobei Lactoferrin in einer Menge zwischen 1 mg und 1000 mg vorhanden ist, bevorzugt zwischen 10 mg und 500 mg, noch stärker bevorzugt zwischen 20 mg und 200 mg.

5. Zusammensetzung nach einem der vorhergehenden Ansprüche, wobei die Zusammensetzung eine medizinische Vorrichtung, ein Nahrungsergänzungsmittel, eine nutrazeutische, diätetische und Ernährungszusammensetzung, ein Lebensmittelprodukt, ein Getränk, ein nutrazeutisches Produkt, ein Medikament, ein medizinisches Lebensmittel, eine pharmazeutische Zusammensetzung oder ein Lebensmittel für spezielle medizinische Zwecke ist.

6. Zusammensetzung nach einem der vorhergehenden Ansprüche zur Verwendung bei der Prävention oder Behandlung von Atemwegserkrankungen.

7. Zusammensetzung nach Anspruch 6 zur Verwendung bei der Prävention oder Behandlung von Rhinitis, allergischer Rhinitis, Rhinopharyngitis oder Erkältung, Rhinosinusitis, Sinusitis, Pharyngitis, Epiglottitis, Laryngitis, Bronchitis, Bronchiolitis und Bronchiektasie.

8. Zusammensetzung nach einem der Ansprüche 1 bis 5 zur Verwendung als Stimulans des Immunsystems.

## Revendications

1. Composition contenant une association d'un extrait de *Pelargonium sidoides* et de lactoferrine en mélange avec un vecteur acceptable approprié.

2. Composition selon la revendication 1 sous forme de composition liquide ou solide à usage oral.

3. Composition selon la revendication 1 ou 2, ledit extrait de *Pelargonium sidoides* étant présent en une quantité comprise entre 1 mg et 1000 mg, de préférence entre 10 mg et 500 mg et encore plus préférablement entre 20 mg et 200 mg.

4. Composition selon l'une quelconque des revendications précédentes, ladite lactoferrine étant présente en une quantité comprise entre 1 mg et 1000 mg, de préférence entre 10 mg et 500 mg et encore plus préférablement entre 20 mg et 200 mg.

5. Composition selon l'une quelconque des revendications précédentes, ladite composition étant un dispositif médical, un complément alimentaire, une composition nutraceutique, diététique et nutritionnelle, un produit alimentaire, une boisson, un produit nutraceutique, un médicament, un aliment médicamenteux, une composition pharmaceutique ou un aliment destiné à des fins médicales spéciales.

6. Composition selon l'une quelconque des revendications précédentes destinée à être utilisée dans la prévention ou le traitement de maladies des voies respiratoires.

7. Composition selon la revendication 6 destinée à être utilisée dans la prévention ou le traitement de la rhinite, de la rhinite allergique, de la rhinopharyngite ou du rhume, de la rhinosinusite, de la sinusite, de la pharyngite, de l'épiglottite, de la laryngite, de la bronchite, de la bronchiolite et de la bronchectasie.

8. Composition selon l'une quelconque des revendications 1 à 5, destinée à être utilisée comme stimulant du système immunitaire.
